# EUROPÄISCHE PATENTSCHRIFT

(11) **EP 1 485 158 B1**
(45) Veröffentlichungstag und Bekanntmachung des Hinweises auf die Patenterteilung: **16.01.2008**
(21) Anmeldenummer: 03744299.3
(22) Anmeldetag: 17.01.2003
(51) Int. Cl.: A61M 25/00

(54) **KATHETER**
CATHETER
SONDE

(30) Priorität: 20.03.2002 DE 10212462; 05.11.2002 DE 10251733; 20.11.2002 DE 10254242; 16.12.2002 DE 10259002
(43) Veröffentlichungstag der Anmeldung: 15.12.2004
(73) Patentinhaber: Sauer, Manfred, 74931 Lobbach (DE)
(72) Erfinder: Sauer, Manfred, 74931 Lobbach (DE)
(74) Vertreter: Naumann, Ulrich
(86) Internationale Anmeldenummer: PCT/DE2003/000129
(87) Internationale Veröffentlichungsnummer: WO 2003/077981

(56) Entgegenhaltungen:
- EP-A- 0 384 476
- EP-A- 0 795 339
- EP-A- 1 149 604
- WO-A-96/33763
- US-A- 3 599 641

## Beschreibung

Die Erfindung betrifft einen Katheter zur Harnableitung mit einem flexiblen Schlauch und einer am einführseitigen Ende des Schlauchs fest angeordneten Einführhilfe zum Einführen in die Harnröhre bzw. zum Durchführen in die Blase, wobei der Schlauch im Bereich vor der Einführhilfe mindestens eine Öffnung aufweist.

Zur Bewältigung der Inkontinenz männlicher Personen, insbesondere bei Querschnittslähmung, wird u.a. der sogenannte intermittierende Selbstkatheterismus (ISK) angewandt, wonach sich die inkontinente männliche Person vier bis sechs Mal am Tag selbst katheterisiert. Um zwischen den Katheterisierungsphasen kontinent zu bleiben, wird üblicherweise vom behandelnden Arzt ein Medikament verordnet, welches die Blase deaktiviert bzw. ruhig stellt.

Bislang bekannte Blasenkatheter zur Ableitung von Urin sind jedoch in der Praxis problematisch, da nämlich beim Einführen des Katheters in die Harnröhre und beim Durchführen der Katheterspitze durch die Harnröhre in die Blase hinein in der Harnröhre gebildete Taschen, Faltungen, Bögen oder dergleichen zu überwinden sind. Schiebt man den Katheter mit entsprechender Kraft gegen die in der Harnröhre vorkommenden Hindernisse, besteht eine ganz erhebliche Verletzungsgefahr.

Lediglich beispielhaft wird zum Stand der Technik auf die EP 0 384 476 B1 verwiesen, wonach nämlich als Einführhilfe eine ganz besondere Katheterspitze vorgesehen ist, die zwar flexibel bzw. elastisch ausgebildet ist, sich jedoch zum freien Ende hin konisch verjüngt und am vorderen bzw. freien Ende abgerundet ist. Aufgrund dieser konkreten Ausgestaltung lässt sich zwar der Katheter unter Überwindung der zuvor genannten Problemstellen durch die Harnröhre hindurch in die Blase einschieben, besteht jedoch aufgrund der erforderlichen Kraftaufwendung auch hier eine ganz erhebliche Verletzungsgefahr.

Der vorliegenden Erfindung liegt daher die Aufgabe zugrunde, einen Katheter, insbesondere einen Einmalkatheter, derart auszugestalten und weiterzubilden, dass er vom Anwender einfach zu handhaben ist und dass die beim Einschieben in die Harnröhre und Durchschieben durch die Harnröhre hindurch übliche Verletzungsgefahr ganz erheblich reduziert ist.

Die zuvor genannte Aufgabe ist durch einen Katheter mit den Merkmalen des Patentanspruches 1 gelöst. Danach ist der gattungsbildende Katheter dadurch gekennzeichnet, dass sich die Einführhilfe mit im wesentlichen dem gleichen Durchmesser an den Schlauch anschließt und mit einem abgerundeten Kopfbereich endet, dessen Durchmesser zumindest geringfügig größer ist als der Durchmesser des Schlauchs.

Erfindungsgemäß ist erkannt worden, dass sich die Einführhilfe, ganz im Gegensatz zu dem bislang bekannten Stand der Technik, im wesentlichen mit gleichem Durchmesser an den Schlauch anschließt und mit einem abgerundeten Kopfbereich endet, wobei dieser einen zumindest geringfügig größeren Durchmesser hat als der Durchmesser der Schlauches. Damit wird erreicht, dass beim Einführen der Katheterspitze in die Harnröhre diese aufgrund der Ausgestaltung des abgerundeten, kugeligen Kopfbereichs zumindest geringfügig gedehnt wird, so dass Taschen, Faltungen und Bögen wesentlich einfacher zu überwinden sind, als dies mit einer sich verjüngenden Spitze der Fall ist. Dabei konnte festgestellt werden, dass der erforderliche Kraftaufwand zum Einschieben des Katheters wesentlich geringer ist als bei herkömmlichen Kathetern, wodurch sich die Verletzungsgefahr abermals ganz erheblich verringert. Letztendlich eignet sich der erfindungsgemäße Katheter hervorragend zur Selbstkatheterisierung, und zwar auch dann, wenn die Handhabung des Katheters aufgrund einer Behinderung ganz erheblich beeinflusst ist.

In vorteilhafter Weise ist die Einführhilfe aus einem ähnlichen Material wie der Schlauch gefertigt, wobei die Einführhilfe insgesamt eine geringere Festigkeit wie der Schlauch aufweist. Im Konkreten könnte die Einführhilfe mindestens halb so fest bzw. steif wie der Schlauch ausgeführt sein, um nämlich das Einführen des Schlauchs mittels der Einführhilfe zu erleichtern und die Verletzungsgefahr dabei zu verringern.

Sowohl die Einführhilfe als auch der Schlauch könnten zum freien Ende hin bzw. zum einführseitigen Ende hin stetig oder in Stufen weicher werden, wobei der Schlauch und/oder die Einführhilfe vom freien Ende her über eine Strecke von 5 bis 10 cm weicher als der übrige Schlauch ausgeführt ist.

Der Schlauch und die Einführhilfe könnten beide aus Kunststoff, insbesondere aus PVC, gefertigt sein. Dabei ist es von ganz besonderem Vorteil, wenn der Schlauch und/oder die Einführhilfe zum freien Ende hin vorzugsweise mittels Weichmacher chemisch behandelt ist, um nämlich zum freien Ende hin stetig weichere Bereiche zu erhalten. Die Behandlung mit Weichmachern begünstigt die Fertigung des Katheters dahingehend, dass sowohl der Schlauch als die Einführhilfe aus PVC bestehen und dass die beiden Teile durch gegenseitige Diffusion von Weichmachern materialschlüssig miteinander verbunden sind.

Zum ungehinderten Abfluss des Urins sind im Schlauch vorzugsweise zwei Öffnungen vorgesehen, wobei diese in weiter vorteilhafter Weise unmittelbar hinter der Einführhilfe ausgebildet sind. Diese Öffnungen werden beispielsweise stanztechnisch hergestellt. Um scharfe Kanten im Bereich der Öffnungen zu vermeiden, sind diese in weiter vorteilhafter Weise zumindest nach außen hin abgerundet, wobei eine solche Abrundung der Öffnung durch Temperaturbehandlung des die Öffnung umfassenden Bereichs realisierbar ist. Dabei erweicht das Material und bildet aufgrund der sich ergebenden Oberflächenspannung eine abgerundete Oberfläche im Bereich des Rands der Öffnung, so dass auch insoweit die Gefahr einer Verletzung vermieden ist.

Auch ist es möglich, zumindest den zum Einführen dienenden Bereich des Schlauchs oder der Einführhilfe so zu behandeln, dass das Benetzungsverhalten gegenüber einem konventionellen Gleitmittel begünstigt ist. Eine solche Behandlung kann auf chemische Weise und/oder mechanisch erfolgen. Mit einer solchen Behandlung wird jedenfalls erreicht, dass das Gleitmittel besser an der Außenwandung des Schlauchs und/oder der Einführhilfe haftet, so dass auch insoweit das Einführen des Schlauchs in die Harnröhre begünstigt ist.

Bei einer kugeligen Einführhilfe sind besondere Öffnungen erforderlich, um nämlich die Körperflüssigkeit aufnehmen zu können. Dazu sind im Schlauch zwei oder mehrere Öffnungen vorgesehen, wobei diese in weiter vorteilhafter Weise unmittelbar hinter der Einführhilfe ausgebildet sind. Die Öffnungen werden beispielsweise stanztechnisch hergestellt. Um scharfe Kanten im Bereich der Öffnungen zu vermeiden, sind diese in weiter vorteilhafter Weise zumindest nach außen hin abgerundet, wobei eine solche Abrundung der Öffnung durch Temperaturbehandlung des die Öffnung umfassenden Bereichs realisierbar ist. Dabei erweicht das Material und bildet aufgrund der sich ergebenden Oberflächenspannung eine abgerundete Oberfläche im Bereich des Rands der Öffnung, so dass auch insoweit die Gefahr einer Verletzung vermieden ist.

Schließlich ist es möglich, zumindest den zum Einführen dienenden Bereich des Schlauchs oder der Einführhilfe so zu behandeln, dass das Benetzungsverhalten gegenüber einem konventionellen Gleitmittel begünstigt ist. Eine solche Behandlung kann auf chemische Weise und/oder mechanisch erfolgen. Mit einer entsprechenden Behandlung wird jedenfalls erreicht, dass das Gleitmittel besser an der Außenwandung des Schlauchs und/oder der Einführhilfe haftet, so dass auch insoweit das Einführen des Schlauchs in die Harnröhre begünstigt ist.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung eines Ausführungsbeispiels der Erfindung anhand der Zeichnung zu verweisen. In Verbindung mit der Erläuterung des bevorzugten Ausführungsbeispiels, der Erfindung anhand der Zeichnung werden auch im allgemeinen bevorzugte Ausgestaltungen und Weiterbildungen der Lehre erläutert. In der Zeichnung zeigt
- Fig. 1: in einer schematischen Ansicht ein erstes Ausführungsbeispiel eines erfindungsgemäßen Katheters mit Kugelspitze und
- Fig. 2: in einer schematischen Ansicht ein zweites Ausführungsbeispiel eines erfindungsgemäßen Katheters mit Kugelspitze, wobei dort Zonen unterschiedlicher Härte angedeutet sind.

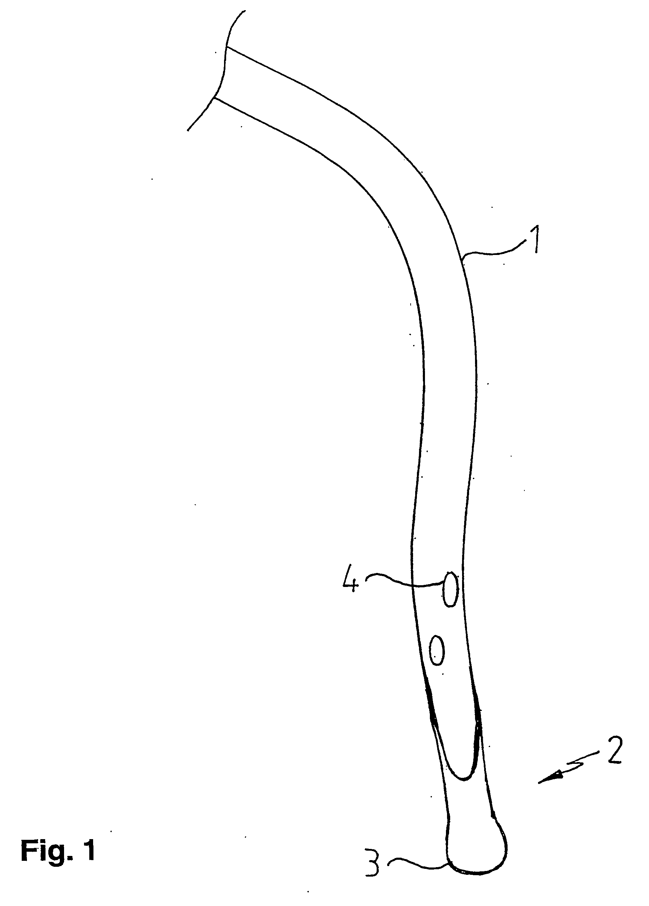

Fig. 1 zeigt ein Ausführungsbeispiel eines erfindungsgemäßen Katheters, wobei der Einfachheit halber lediglich der flexible Schlauch 1 mit der Einführhilfe 2 gezeigt ist. Die Einführhilfe 2 ist am einführseitigen Ende des Schlauchs 1 angeordnet bzw. dort fest mit dem Schlauch 1 verbunden. Letztendlich handelt es sich bei der Einführhilfe 2 um einen integralen Bestandteil des Schlauchs 1, wobei die Einführhilfe 2 wesentlich weicher ist als der Schlauch 1.

Erfindungsgemäß hat die Einführhilfe 2 einen im wesentlichen gleichen Durchmesser wie der Schlauch 1 und schließt sich entsprechend an den Schlauch 1 - materialschlüssig - an. Der Kopfbereich 3 der Einführhilfe 2 ist kugelig ausgeführt und hat einen zumindest geringfügig größeren Durchmesser als der Schlauch 1. Dadurch ergeben sich die zuvor bereits erörterten Vorteile.

Fig. 1 zeigt des Weiteren, dass der Schlauch 1 zur Harnableitung mit Öffnungen 4 ausgestattet ist, wobei bei dem hier gewählten Ausführungsbeispiel insgesamt zwei Öffnungen 4 vorgesehen sind. Zum schnelleren Hamabfluss können auch mehrere Öffnungen 4 vorgesehen sein. Die umlaufenden Kanten der Öffnungen 4 sind abgerundet, um die Gefahr einer Verletzung beim Hindurchschieben durch die Harnröhre weitestgehend auszuschließen.

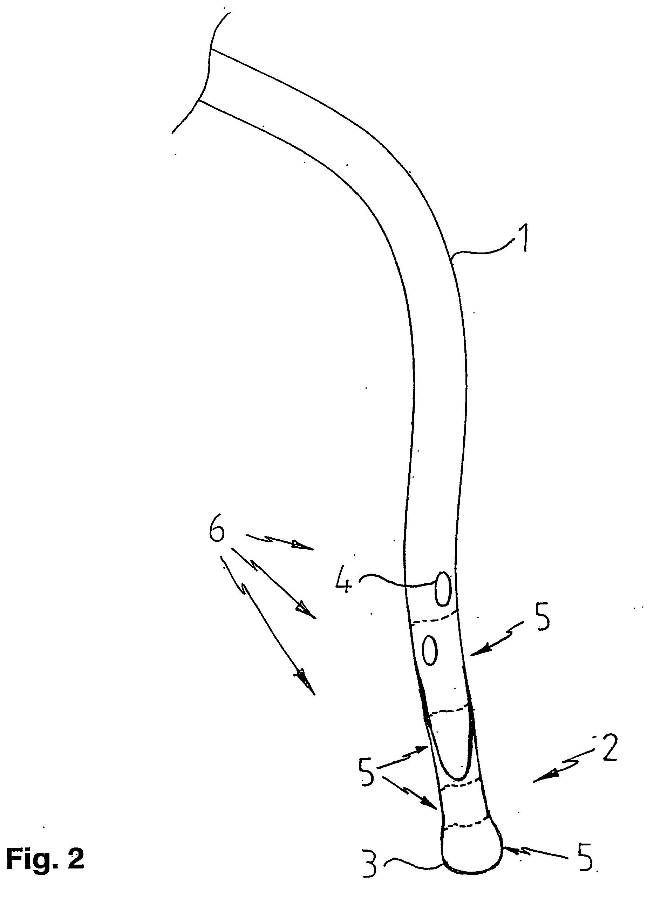

Bei dem in Fig. 2 gezeigten weiteren Ausführungsbeispiel sind die Einführhilfe 2 und das einführseitige Ende des Schlauchs 1 mit unterschiedlich weichen Zonen 5 ausgebildet, so dass ein sanftes Einführen des Katheters abermals begünstigt ist. Außerdem ist in Fig. 2 ein Bereich 6 angedeutet, an dem durch chemische Behandlung ein Gleitmittels besonders gut haftet.

## Patentansprüche

1. Katheter zur Harnableitung mit einem flexiblen Schlauch (1) und einer am einführseitigen Ende des Schlauchs (1) fest angeordneten Einführhilfe (2) zum Einführen in die Harnröhre bzw. zum Durchführen in die Blase, wobei der Schlauch (1) im Bereich vor der Einführhilfe (2) mindestens eine Öffnung (4) aufweist,
**dadurch gekennzeichnet, dass** sich die Einführhilfe (2) mit im wesentlichen dem gleichen Durchmesser an den Schlauch (1) anschließt und mit einem abgerundeten Kopfbereich (3) endet, dessen Durchmesser zumindest geringfügig größer ist als der Durchmesser des Schlauchs (1).

2. Katheter nach Anspruch 1, **dadurch gekennzeichnet, dass** die Einführhilfe (2) aus einem ähnlichen Material wie der Schlauch (1) gefertigt ist, jedoch eine geringere Festigkeit wie der Schlauch (1) aufweist und/oder dass die Einführhilfe (2) mindestens nur halb so fest bzw. steif wie der Schlauch (1) ist und/oder dass die Einführhilfe (2) zum freien Ende hin stetig oder in Stufen weicher wird und/oder dass der Schlauch zu seinem einführseitigen Ende hin stetig oder in Stufen weicher wird.

3. Katheter nach Anspruch 1 oder 2, **dadurch gekennzeichnet, dass** der Schlauch und/oder die Einführhilfe zum freien Ende hin vorzugsweise mittels Weichmacher chemisch behandelt ist und/oder dass der Schlauch (1) aus PVC gefertigt ist und dass die Einführhilfe (2) durch gegenseitige Diffusion von Weichmachern materialschlüssig mit dem Schlauch (1) verbunden ist.

4. Katheter nach einem der Ansprüche 1 bis 3, **dadurch gekennzeichnet, dass** im Bereich vor der Einführhilfe (2) zwei oder mehrere Öffnungen (4) vorgesehen sind und dass die Öffnung bzw. Öffnungen (4) vorzugsweise stanztechnisch hergestellt ist bzw. sind, wobei die Öffnungen (4) zumindest nach außen hin abgerundete Kanten aufweisen können, die durch Temperaturbehandlung des die Öffnungen (4) umfassenden Bereichs realisiert sind.

5. Katheter nach einem der Ansprüche 1 bis 4, **dadurch gekennzeichnet, dass** der Bereich der Einführhilfe (2) und/oder das einführseitige Ende des Schlauchs (1) und/oder der zum Einführen in die Harnröhre dienende Teil des Schlauchs (1) zur Begünstigung des Benetzungsverhaltens gegenüber Gleitmitteln mechanisch und/oder chemisch behandelt ist.

## Claims

1. Catheter for urine removal, having a flexible tube (1) and, arranged securely at the introduction end of the tube (1), an introduction aid (2) for introduction into the urethra or for passing through into the bladder, the tube (1) having at least one opening (4) in the region upstream of the introduction aid (2), **characterised in that** the introduction aid (2), having substantially the same diameter, adjoins the tube (1) and terminates with a rounded head region (3), the diameter of which is at least slightly greater than the diameter of the tube (1).

2. Catheter according to claim 1, **characterised in that** the introduction aid (2) is manufactured from a similar material to that of the tube (1) but has a lesser strength than the tube (1) and/or **in that** the introduction aid (2) is at least only half as strong or rigid as the tube (1) and/or in that the introduction aid (2) becomes softer continuously or in stages towards the free end and/or **in that** the tube becomes softer continuously or in stages towards its introduction end.

3. Catheter according to claim 1 or 2, **characterised in that** the tube and/or the introduction aid is/are treated chemically preferably by means of plasticisers towards the free end and/or **in that** the tube (1) is manufactured from PVC and **in that** the introduction aid (2) is connected to the tube (1) integrally in terms of material by mutual diffusion of plasticisers.

4. Catheter according to any one of claims 1 to 3, **characterised in that** two or more openings (4) are provided in the region upstream of the introduction aid (2) and **in that** the opening or openings (4) is/are manufactured preferably by punching technology, it being possible for the openings (4) to have edges that are rounded at least towards the outside and that are produced by temperature treatment of the region comprising the openings (4).

5. Catheter according to any one of claims 1 to 4, **characterised in that** the region of the introduction aid (2) and/or the introduction end of the tube (1) and/or the portion of the tube (1) used for introduction into the urethra is/are treated mechanically and/or chemically in order to promote wetting behaviour with respect to lubricants.

## Revendications

1. Cathéter pour la dérivation d'urine avec un tuyau (1) flexible et une aide d'introduction (2) disposée de manière fixe à l'extrémité du côté introduction du tuyau (1) pour l'introduction dans l'urètre ou respectivement pour le passage dans la vessie, le tuyau (1) présentant au moins une ouverture (4) dans la zone de l'aide d'introduction (2),
**caractérisé par le fait que** l'aide d'introduction (2) se raccorde au tuyau (1) à essentiellement le même diamètre et se termine par une zone de tête (3) arrondie dont le diamètre est au moins légèrement plus grand que le diamètre du tuyau (1).

2. Cathéter selon la revendication 1, **caractérisé par le fait que** l'aide d'introduction (2) est fabriquée en un matériau analogue à celui du tuyau (1) mais présente une fermeté plus faible que le tuyau (1) et/ou que l'aide d'introduction (2) est seulement moitié au moins aussi ferme ou respectivement rigide que le tuyau (1) et/ou que l'aide d'introduction (2) devient plus molle continuellement ou par paliers en direction de l'extrémité libre et/ou que le tuyau devient plus mou continuellement ou par paliers en direction de son extrémité du côté introduction.

3. Cathéter selon la revendication 1 ou 2, **caractérisé par le fait que** le tuyau et/ou l'aide d'introduction sont traités chimiquement, de préférence au moyen d'un plastifiant, en direction de l'extrémité libre et/ou que le tuyau (1) est fabriqué en PVC et que l'aide d'introduction (2) est reliée au tuyau (1) à fermeture de matière par diffusion mutuelle de plastifiants.

4. Cathéter selon l'une des revendications 1 à 3, **caractérisé par le fait que** deux ouvertures (4) ou plus sont prévues dans la zone avant l'aide d'introduction (2) et que l'ouverture ou respectivement les ouvertures (4) est ou respectivement sont fabriquée(s) par une technique de poinçonnage, les ouvertures (4) pouvant présenter des arêtes arrondies au moins vers l'extérieur qui sont réalisées par traitement thermique de la zone comprenant les ouvertures (4).

5. Cathéter selon l'une des revendications 1 à 4, **caractérisé par le fait que** la zone de l'aide d'introduction (2) et/ou l'extrémité du côté introduction du tuyau (1) et/ou la partie du tuyau (1) servant pour l'introduction dans l'urètre sont traités mécaniquement et/ou chimiquement pour favoriser le comportement au mouillage par rapport à des lubrifiants.
